# EUROPÄISCHE PATENTANMELDUNG

(11) **EP 3 861 966 A1**
(43) Veröffentlichungstag der Anmeldung: **11.08.2021**
(21) Anmeldenummer: 20401036.7
(22) Anmeldetag: 05.06.2020
(51) Int. Cl.: A61F 2/54, A61F 2/58, A61F 2/76

(54) **ARMPROTHESE**

(30) Priorität: 06.02.2020 DE 102020103112
(71) Anmelder: Stamos + Braun Prothesenwerk GmbH, 01307 Dresden (DE)
(72) Erfinder: Braun, Christoph, 01307 Dresden (DE)
(74) Vertreter: Weissfloh, Ingo

(57) **Zusammenfassung**

Der Erfindung liegt die Aufgabe zugrunde, eine Armprothese zu schaffen, die sich durch einen einfachen modularen Aufbau auszeichnet, extrem leicht ist und einfach nachjustierbar ist.

Die Armprothese in Leichtbauweise besitzt eine hohe Stabilität und bei einem geringeren Gewicht gegenüber bisher bekannten Lösungen, bei der am distalen Ende eines an sich bekannten Schaftes (1) ein Loch als Aufnahme für einen Verbinder (3) in Form einer Gewindestange (3) angeordnet ist und die Gewindestange (3) innerhalb des Schaftes (1) in einem auf der Gewindestange (3) aufgeschraubten Innenadapter (4) endet, wobei der Innenadapter (4) auf der Seite, die an der Schaftinnenseite anliegt, dem Schaft (1) um das Loch herum angepasst ist und auf der Gewindestange (3) außerhalb des Schaftes (1) eine Kontermutter (5) bzw. ein Außenadapter (5) angeordnet ist, dessen Seite zum Schaft (1) als Halbschale (6) ausgeführt und am anderen Ende des Verbinders (3) ein Ellbogengelenk (7) angeordnet ist, welches an beiden Enden ein Innengewinde für die Aufnahme je eines Verbinders (3), den Verbinder (3) vom Schaft (1) und einen Verbinder (3) zu einem Handgelenk (8), besitzt und das Handgelenk (8) an einer Seite ein Innengewinde zur Aufnahme des Verbinders (3) vom Ellbogengelenk (7) besitzt und am anderen Ende das Handgelenk (8) einen Ansatz (17) mit einem Außengewinde für die Aufnahme einer Handprothese (19) besitzt, wobei alle Einzelteile der Armprothese aus Kunststoff bestehen.

## Beschreibung

Armprothese in Leichtbauweise bestehend aus Schaft, Ellbogengelenk, Handgelenk, Handprothese, Verbindern zwischen den Gelenken und dem Schaft und Adaptern am Schaft.

Die Verbindungselemente zwischen den Prothesenteilen müssen justierbar ausgeführt sein, um eine individuelle Anpassung am jeweiligen Prothesenträger vornehmen zu können. In der Schrift DE 19 22 619 wird ein justierbares Verbindungselement zwischen Prothesenteilen unter Verwendung eines Kugelgelenkes beschrieben. Eine auf das eine Prothesenteil aufgesetzte Kalotte mit einer auf ihr zentrisch auf der Spitze stehenden vierflächigen Pyramide und einer am anderen Prothesenteil angeordneten Kugelpfanne ist mit den konkav ausgebildeten Pyramidenflächen zusammenwirkenden Stellschrauben, die nur in zwei senkrecht aufeinanderstehenden Ebenen, in den die Kunstgliedachse liegt, verstellbar. Die Pyramide und die Kugelpfanne lassen sich nur in zwei definierten Ebenen verschwenken und je nach der im Einzelfall gewünschten Winkelstellung festlegen lassen. Auf diese Weise lässt sich die Justierung einer Armprothese auf die anatomische Winkelstellung abstimmen.

In der Schrift DE 27 11 551 C2 wird eine Bein- oder Armprothese mit dem lasttragenden Teil aus mit Kohlenstofffasern verstärktem Kunststoff beschrieben, um bei einer gegebenen Lastaufnahmefähigkeit die Bein- oder Armprothese in einem geringeren Gewicht als bisher herzustellen. Dazu wird vorgeschlagen, dass der lasttragende Teil aus einem Rahmenwerk besteht, welches einen Hohlraum umschließt und aus starren, langestreckten, sich axial zum lasttragenden Teil erstreckenden Elementen und sich diagonal zu den axial verlaufenden Elementen erstreckenden Elementen gebildet ist und dass die Elemente an denjenigen Stellen, an denen sie sich kreuzen, fest miteinander verbunden sind. Weiterhin sind weitere, reifenartig ausgebildete Elemente etwa rechtwinklig zu den axial verlaufenden Elementen möglich. An diesem Rahmenwerk sind Funktionseinheiten gehalten.

Aus der Schrift DE 10 2006 046 927 A1 ist unter anderem zu entnehmen, dass der Schaft sich aus einen Innen- und Außenschaft zusammensetzt. Der Außenschaft besitzt ein distales Endstück mit einem Anschluss für wenigstens ein künstliches Glied, z. B. ein Modulrohr als Teil eines künstlichen Beins. Dieses Modulrohr ist fest mit dem Außenschaft verbunden. Die gleiche Aussage ist aus der Schrift DE 10 2006 046 928 B4 zu entnehmen.

In einer weiteren Schrift, der DE 20 2011 000 316 U1 wird ein textilbedeckter, polymerer Liner, der ausgelegt ist, um als eine eigenständige Trennschicht zwischen einem Restglied eines Amputierten und dem Inneren eines prothetischen Schaftes zu fungieren. Diese Erfindung betrifft insbesondere einen solchen prothetischen Liner mit begrenzten und kontrollierten Längsdehnungseigenschaften. Die Liner der vorliegenden Erfindung können Verriegelungsliner sein. Demzufolge kann ein Liner der vorliegenden Erfindung ein Ankopplungselement an seinem geschlossenen Ende umfassen. Das Ankopplungselement kann verwendet werden, um den Liner an dem Schaftabschnitt einer Prothese zu befestigen. Eine bestimmte Ausführungsform eines Ankopplungselements der vorliegenden Erfindung umfasst eine metallische T-Gewindemutter, die an dem offenem Ende der dehnungsregulierenden Textilie des Liners durch einen überlappenden und im Wesentlichen einhüllenden halbflexiblen Schirm befestigt ist. Der T-Mutter-Abschnitt des Ankopplungselements umfasst einen Basisabschnitt, von dem sich ein hohler Ansatz mit Innengewinde erstreckt. Vorzugsweise ist wenigstens die innere Oberfläche des Basisabschnitts der T-Mutter konkav. So dass sie besser an die gerundete distale Form des Liners passt, wenn darin ein Restglied eingeführt ist. Der Gewindeansatz ist vorgesehen, um einen mit einem entsprechenden Gewinde versehenen Stift, einen Lanyard-Verbinder oder ein anderes Verbindungselement aufzunehmen, das verwendet werden kann, um den Liner an einer passenden Ankopplungskomponente zu befestigen, die zu dem Schaft einer Prothese gehört. Der umhüllende Schirmabschnitt des Ankopplungselements kann aus Polyurethan bestehen, das sich mit der Textilie an dem geschlossenen Ende des Liners verbindet und die dort positionierte T-Gewindemutter sichert. Es kann auch möglich sein, einen harten Silikongummi oder ein weiteres, ähnliches Material für den Schirm zu ersetzen. Das Design dieses Schirms unterscheidet sich von bekannten Designs dahingehend, dass dieser Schirm vorzugsweise einen akkordionartigen Aufbau besitzt. Das heißt, der Schirm hat eine gestufte Konfiguration, die ein leichtes Zusammenfalten oder Zusammendrücken des Schirms erlaubt, wenn das Gewicht eines Amputierten das geschlossene Ende eines zugehörigen Liners in den Boden des Prothesenschafts drückt.

Der Erfindung liegt die Aufgabe zugrunde, eine Armprothese zu schaffen, die sich durch einen einfachen modularen Aufbau auszeichnet, extrem leicht ist und einfach nachjustierbar ist.

Die Erfindungsaufgabe wird durch eine Armprothese mit den Merkmalen des Hauptanspruchs und den dazugehörigen Unteransprüchen gelöst.

Die erfindungsgemäße Armprothese besteht aus einem an sich bekannten Schaft für die Aufnahme eines Restgliedes, z. B. eines Oberarmstumpfes oder eines Unterarmstumpfes. Der Schaft besteht aus einem dem jeweiligen Stumpf angepassten Kunststoffhohlkörper. Am distalen Ende des Schaftes ist in diesen Kunststoffkörper eine Ausbuchtung für die Aufnahme eines Innenadapters vorgesehen. Dies wird im Herstellungsprozess durch einen Dummy erreicht, der nur zur Herstellung der Ausbuchtung benutzt wird und nicht im Schaft verbleibt. Der Schaft besitzt in dem Bereich der Ausbuchtung ein mittig liegendes Loch. Dieses Loch wird bei der Herstellung des Schaftes als durchgehendes Loch für einen Verbinder herausgearbeitet. Der Verbinder besitzt die Form einer Gewindestange aus Kunststoffvollmaterial oder in einer Vorzugsvariante als Kunststoffrohr mit einer innen liegenden kohlenstofffaserverstärktem Kunststoffschicht bzw. Kunststoffrohr. Diese Variante besitzt trotz geringer Abmaße eine hohe Stabilität und Flexibilität.

Die Gewindestange innerhalb des Schaftes endet in einem Innenadapter, d. h. der Innenadapter ist auf der Gewindestange aufgeschraubt und füllt die Ausbuchtung des Schaftes aus, d. h. der Armstumpf reicht bis maximal an die proximale Seite des Innenadapters. Der Innenadapter ist auf der Seite, die an der Schaftinnenseite um das Loch des Durchgangs des Verbinders anliegt, angepasst. Auf dem Verbinder außerhalb des Schaftes ist eine Art Kontermutter als Außenadapter angeordnet, die der Befestigung des Verbinders am Schaft dient. Der Außenadapter besitzt dazu eine Art Halbschale in Richtung Schaft. Beide Adapter besitzen vorteilhaft an den Seiten der Schaftberührung eine elastische Oberfläche, einen entsprechenden rutschhemmenden, elastischen Überzug oder aufgetragene, elastische Flächen oder einen in einer Nut eingelegten Gummiring, der mit seiner Oberfläche aus der Nut etwas herausragt.

Diese konstruktive Lösung mit einem Innen- und Außenadapter auf der Gewindestange mit ihren angepassten Oberflächen zum Schaft ermöglicht eine Justierbarkeit in alle Richtungen nach vorn und nach hinten und zu den Seiten und gleichzeitig einen sicheren Halt der eingestellten Justierung.

Es ist jedoch auch denkbar, dass der Dummy als Verstärkerelement am oder im Schaft verbleibt. Hierzu muss jedoch die Ausbuchtung des Schaftes vergrößert werden, da der Innenadapter noch hinter dem Schaftbereich mit dem Dummy Platz finden muss.

Die Adapter am Schaft und die Gelenke werden mittels Sicherungsschrauben in Form von z. B. Innensechskantschrauben gesichert. Dies erfolgt durch Bohrungen durch diese Bauteile in Richtung der Verbinder. Vorteilhaft werden diese Bohrungen durch in die Bohrungen eingearbeitete Messinggewindehülsen verstärkt.

Am anderen Ende des Verbinders vom Schaft ist ein Ellbogengelenk angeordnet. Dieses Ellbogengelenk besitzt an beiden Enden ein Innengewinde zur Aufnahme je eines Verbinders, dem Verbinder vom Schaft und einem weiterführenden Verbinder zum Handgelenk. Das Handgelenk besitzt ein Innengewinde zur Aufnahme des Verbinders vom Ellbogengelenk und auf der anderen Seite einen Ansatz mit Außengewinde zur Aufnahme einer Handprothese.

Alle diese Teile, Schaft mit oder ohne Dummy, Innen- und Außenadapter, Ellbogengelenk, Handgelenk und Handprothese bestehen aus Kunststoff und stellen durch ihre konstruktive Lösung gegenüber dem bekannten Stand der Technik eine Art Leichtbauweise dar und gewähren eine hohe Stabilität und Flexibilität.

Es ist auch denkbar, dass der Schaft einem Unterarmstumpf angepasst werden kann. Damit entfallen dann der obere Verbinder und das Ellbogengelenk.

Vorteilhafte Ausführungsbeispiele der Erfindung werden anhand der Zeichnungen näher erläutert. Es zeigen:
Fig. 1 die Draufsicht auf einen Dummy für einen Oberarmschaft,
Fig. 2 die perspektivische Ansicht eines Dummy für einen Oberarmschaft,
Fig. 3 die Seitenansicht eines Dummy für einen Oberarmschaft,
Fig. 4 die Draufsicht auf einen Innenadapter am Oberarm,
Fig. 5 die perspektivische Ansicht eines Innenadapters am Oberarm,
Fig. 6 die Seitenansicht eines Innenadapters am Oberarm,

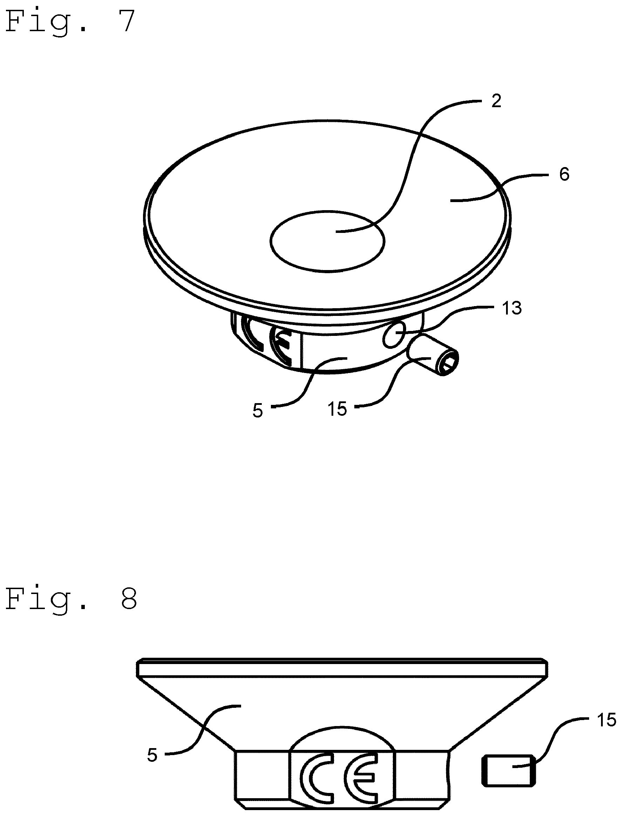

Fig. 7 die perspektivische Ansicht eines Außenadapters am Oberarm,
Fig. 8 die Seitenansicht eines Innenadapters am Oberarm,
Fig. 9 die perspektivische Ansicht eines Dummy für einen Unterarmschaft,
Fig. 10 die Seitenansicht eines Dummy für einen Unterarmschaft,

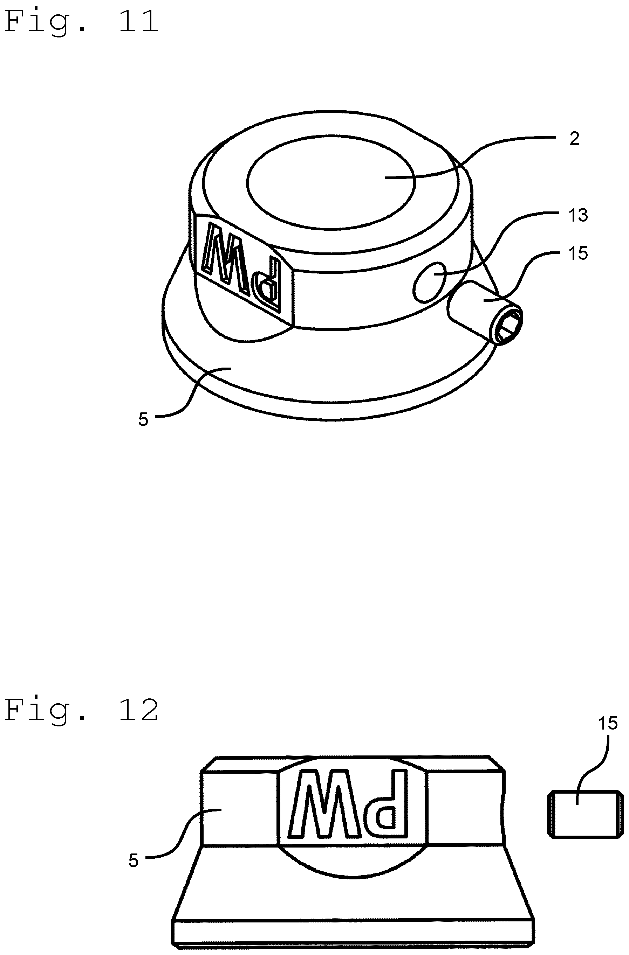

Fig. 11 die perspektivische Ansicht eines Außenadapters am Unterarm,
Fig. 12 die Seitenansicht eines Außenadapters am Unterarm,

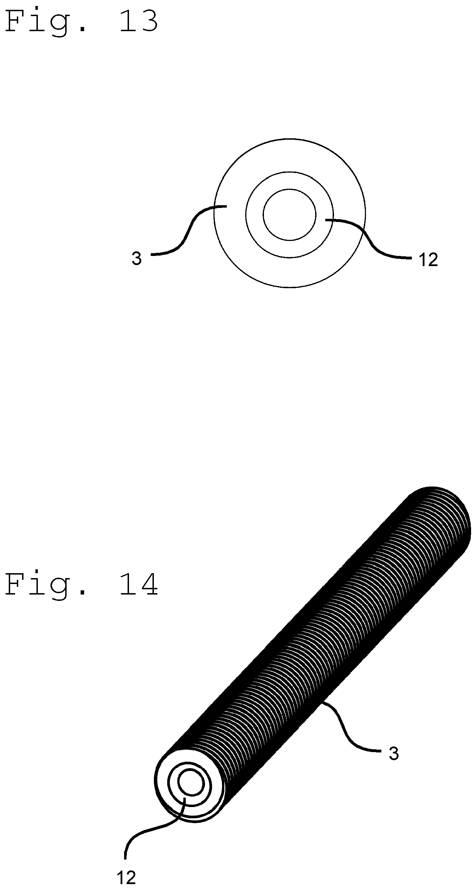

Fig. 13 eine Gewindestange (Verbinder) in Rohrform im Schnitt,
Fig. 14 eine Gewindestange (Verbinder) in perspektivischer Ansicht,

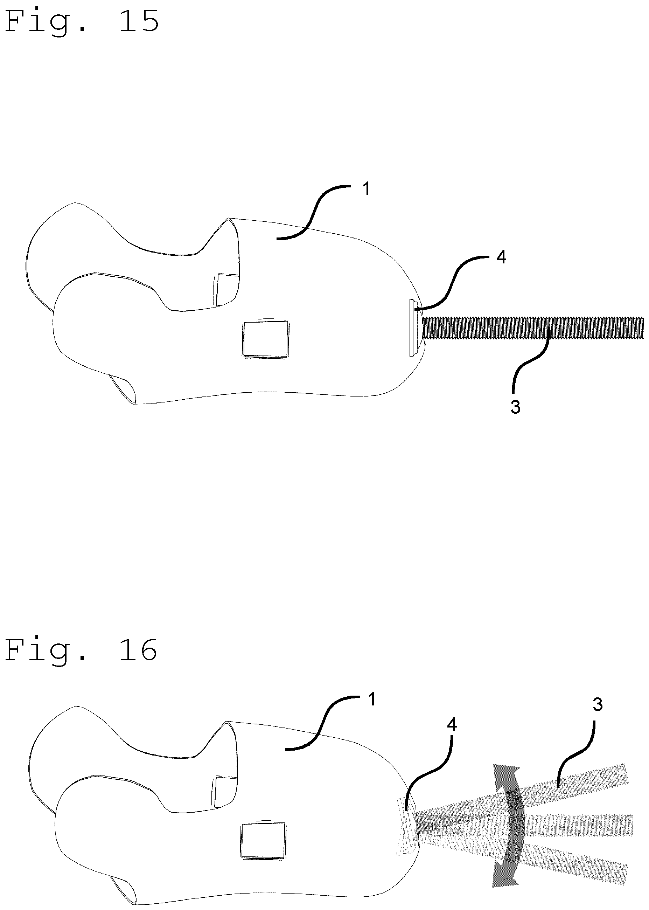

Fig. 15 ein Schaft mit Gewindestange und Innenadapter,
Fig. 16 ein Schaft mit der angedeuteten Beweglichkeit und unterschiedlicher Justierung,
Fig. 17 ein Ellbogengelenk in perspektivischer Ansicht,
Fig. 18 die Seitenansicht eines Ellbogengelenks,
Fig. 19 die Seitenansicht eines Handgelenks,
Fig. 20 die perspektivische Ansicht eines Handgelenks,
Fig. 21 die Schnittdarstellung durch eine Gewindestange (Verbinder) in Rohrform mit innenliegender
kohlenstofffaserverstärktem Kunststoffschicht,
Fig. 22 die Schnittdarstellung eines Gelenkteiles mit einer Messinggewindehülse in der Bohrung für eine Sicherungsschraube und

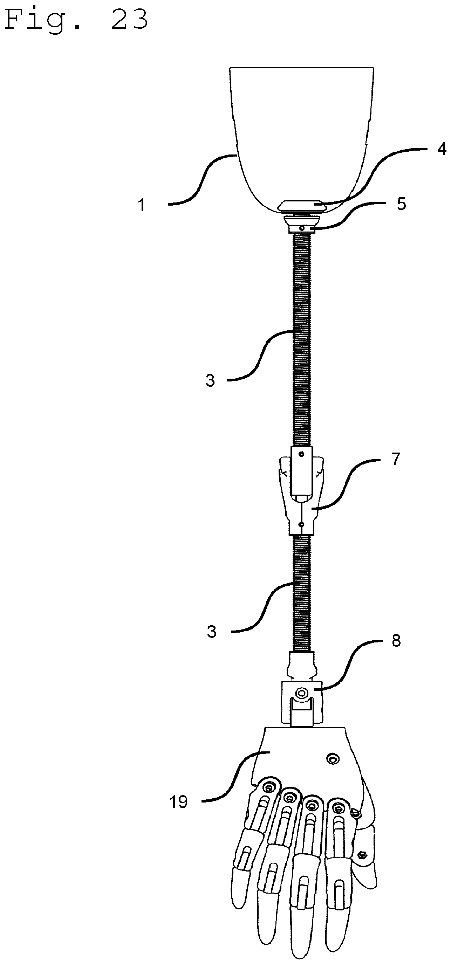

Fig. 23 eine komplette Armprothese mit Schaft, Adaptern, Gelenken, Verbindern und einer Handprothese.

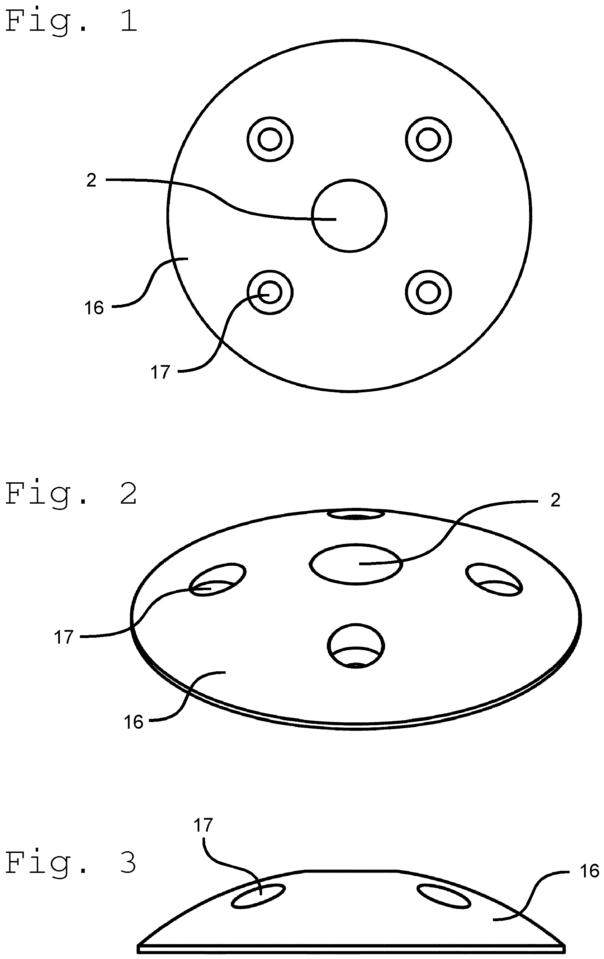

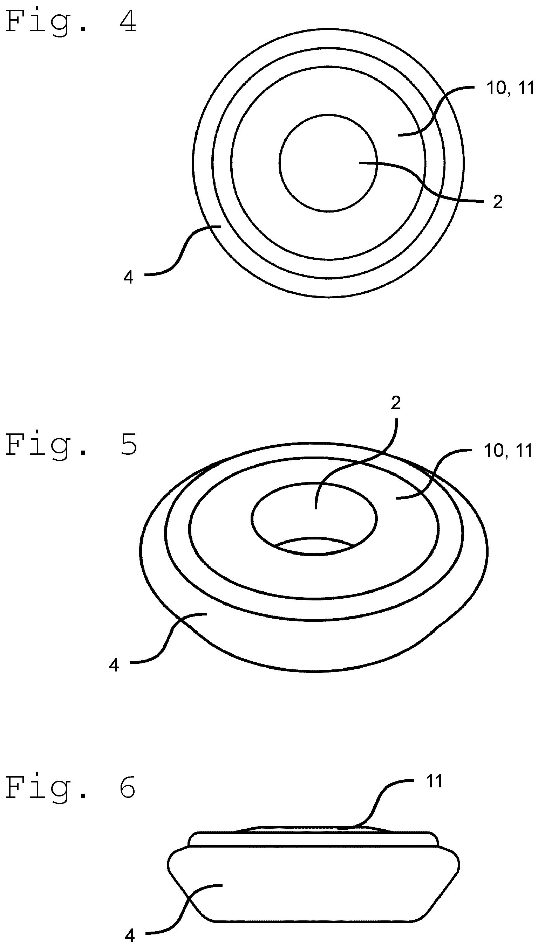

Der erfindungsgemäße Schaft 1 besitzt am distalen Ende eine Ausbuchtung für die Aufnahme eines Innenadapters 4 mit einem Verbinder 3. Die in den Figuren 1 bis 3 und 9 und 10 dargestellten Dummys 16 werden zur Herstellung der Ausbuchtungen am jeweiligen Schaft 1 benutzt und sind im Normalfall nicht Bestandteil des Schaftes 1 als Endprodukt. Es ist jedoch denkbar, dass diese Dummys 16 als Verstärkungselement 16 im Schaft 1 verbleiben. Dieses Verstärkungselement 16 oder Dummy 16, wird während des Tiefzieh- bzw. Gießvorganges in den Schaft 1 eingearbeitet und besitzt bereits eine Öffnung 2 für die spätere Aufnahme 2 einer Gewindestange 3 bzw. Verbinder 3. Die in den Figuren 1 bis 3 und 9 und 10 dargestellten Durchgangslöcher 17 besitzen eine wichtige Funktion bei der Herstellung des Schaftes 1. Der Dummy 16 wird mittels 2-Komponenten-Knetsilikon auf dem Schaft fixiert, auch die Durchgangslöcher 17 werden hierzu mit dem Knetsilikon ausgefüllt und die Oberfläche glatt verarbeitet. Wie bereits oben beschrieben, erfolgt dann der Tiefzieh- bzw. Gießvorgang. Die Öffnung 2 des Dummy 16 wird dann später frei gearbeitet und bietet die Aufnahme 2 für die Gewindestange 3. Innerhalb des Schaftes 1 wird dazu später der Innenadapter 4 bis zum Anschlag aufgeschraubt. In der Aufnahme 2 des Innenadapters 4 ist dafür ein Innengewinde vorgesehen, in den Figuren 4 bis 6 nicht dargestellt. Die Oberfläche des Innenadapters 4 in Richtung Schaft 1 ist der Oberfläche des Schaftes 1 angepasst. Außerhalb des Schaftes 1 ist auf dem Verbinder 3 eine Kontermutter 5 in Form eines Außenadapters 5 vorgesehen. Zur besseren Fixierung des Innenadapters 4 und des Außenadapters 5 am Schaft 1 ist einmal der Außenadapter 5 halbschalenförmig in Richtung Schaft 1 ausgebildet. Diese Halbschale 6 kann eine elastische Oberfläche besitzen, mit einem elastischen Überzug versehen sein oder elastische Teilflächen besitzen. Der Innenadapter 4 kann auf der Oberfläche in Richtung Schaft 1 ebenso gestaltet sein. Vorteilhaft besitzt er jedoch auf dieser Seite zum Schaft 1 eine Nut 10 mit einem darin eingelegten elastischen Ring 11. Dieser Ring 11 ragt etwas über die Oberfläche des Innenadapters 4 hinaus, wie in Figur 6 dargestellt.

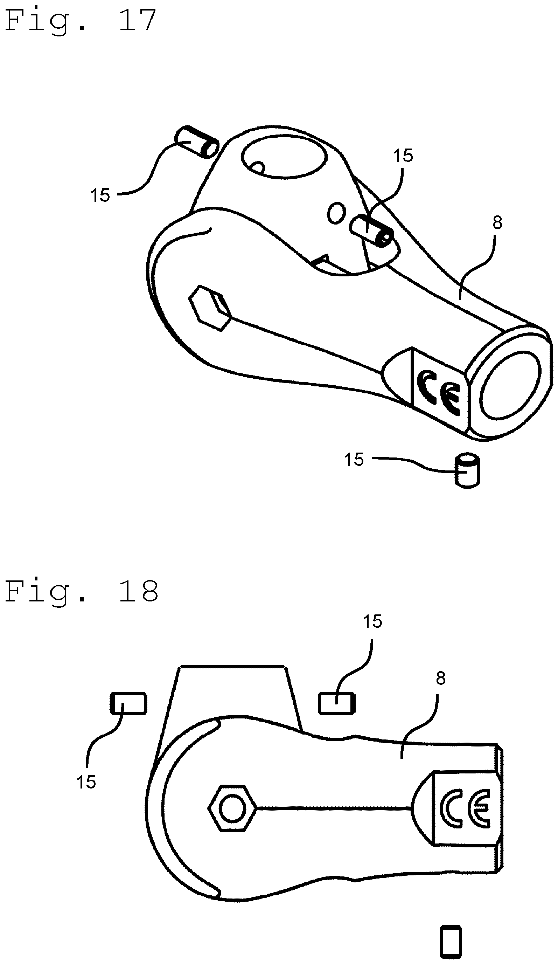

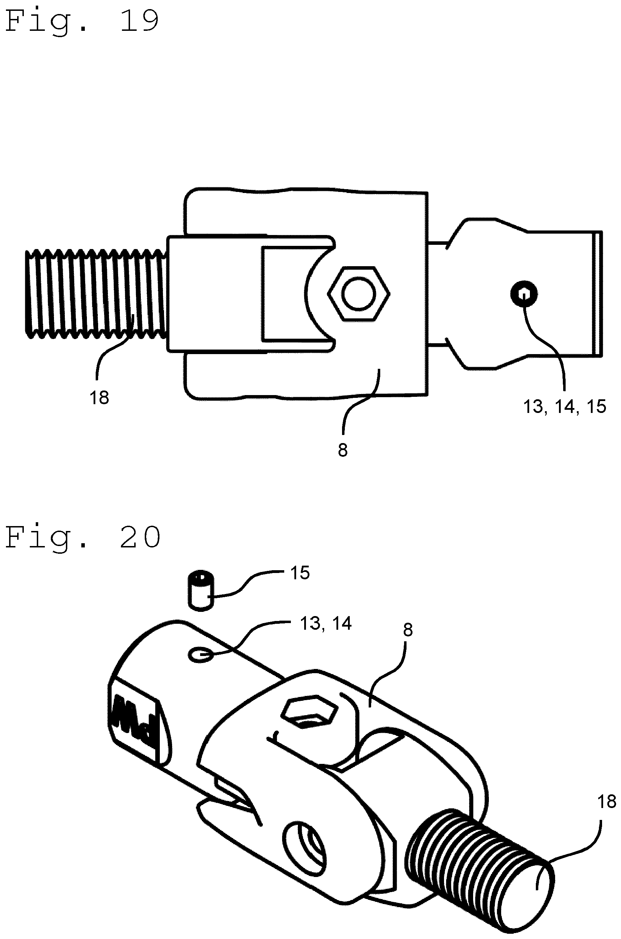

Am anderen Ende des Verbinders 3, vom Schaft 1 aus gesehen, ist ein Ellbogengelenk 7 angeordnet. Das Ellenbogengelenk 7 besitzt, wie in Figur 17 und 18 dargestellt, an beiden Enden ein Innengewinde für die Aufnahme je eines Verbinders 3, den Verbinder 3 vom Schaft 1 und einen Verbinder 3 zu einem Handgelenk 8. Das Handgelenk 8 besitzt, wie in Figur 19 und 20 dargestellt an einer Seite ein Innengewinde zur Aufnahme des Verbinders 3 vom Ellbogengelenk 7 und am anderen Ende des Handgelenks 8 einen Ansatz 18 mit einem Außengewinde für die Aufnahme einer Handprothese 19.

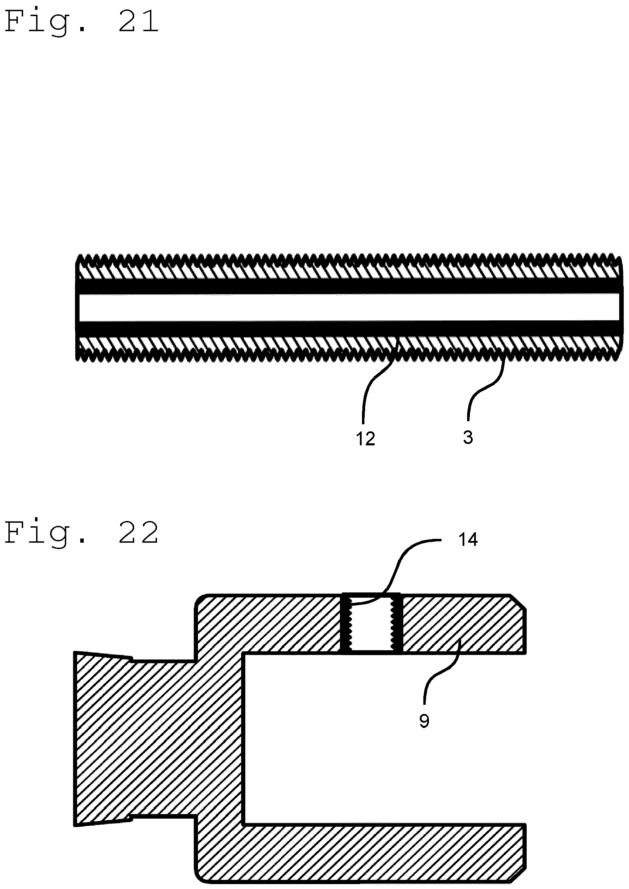

Die Verbindungen zwischen den Adaptern 4 und 5 zu den Verbindern 3 und der Gelenke 7 und 8 zu den Verbindern 3 werden vorteilhaft durch in die Bauteile Adapter 4 und 5 und Gelenke 7 und 8 eingearbeitete Bohrungen 13 und darin eingearbeitete Messinggewindehülsen 14 und durch entsprechende Sicherungsschrauben 15, vorzugsweise durch Innensechskantschrauben 15 gesichert. Diese Messinggewindehülsen 15 sichern eine lange Haltbarkeit gerade der sehr belasteten Gewinde bei eventuell notwendigen Nachjustierungen an der Armprothese. Diese Ausführungsart mit einer Messinggewindehülse 14 ist in Figur 22 an einer Schnittdarstellung eines Gelenkteiles 9 dargestellt.

Alle Einzelteile der Armprothese bestehen aus Kunststoff. So auch die Verbinder 3 aus Vollmaterialstäben mit aufgearbeitetem Gewinde. Es hat sich jedoch als vorteilhaft erwiesen, dass die Verbinder 3 als Rohr ausgebildet sind und auf der Innenseite der Rohre eine Schicht 12 oder ein Rohr 12 aus kohlenstofffaserverstärktem Kunststoff aufgetragen bzw. eingearbeitet ist. Durch diese konstruktive Lösung erhöht sich die Stabilität und Flexibilität der Armprothese auch bei einer weiteren Minimierung des Materialeinsatzes.

Es ist logisch, dass die beschriebene Lösung für eine komplette Armprothese auch reduziert auf eine Unterarmprothese anwendbar ist.

Die Herstellung eines Schaftes 1 nach der erfindungsgemäßen konstruktiven Lösung für die Aufnahme eines Verbinders 3 mit einem Innenadapter 4 und einem Außenadapter 5 wird vorteilhaft mit folgenden Verfahrensschritten durchgeführt. Vom gesunden Arm werden die Maße aufgenommen, danach wird der Armstumpf mit Frischhaltefolie umwickelt und anschließend werden die Umfangsmaße des Armstumpfes aufgenommen. Weiterhin wird anschließend der Armstumpf mit Gipsbinden umwickelt und dieses Wickelmuster nach dem Aushärten vom Armstumpf abgenommen und mit Gips gefüllt. Nach dem Aushärten dieser Gipsfüllung wird diese Gipsform modelliert und ein Innenschaft, z. B. durch ein Tiefziehverfahren, erzeugt. Auf diesen Innenschaft wird ein Dummy 16 aufgesetzt und mittels Knetsilikon am Innenschaft verklebt und die Übergänge werden mit dem Knetsilikon ausgeglichen und die Durchgangslöcher 17 des Dummys 16 ausgefüllt. Anschließend erfolgt unter Nutzung des Innenschaftes mit dem aufgesetzten Dummy 16 das Tiefziehen und Entformen eines Schaftes 1. Nun erfolgt die Einarbeitung eines Lochs in den Schaft 1 am distalen Ende und die Einführung eines Verbinders 3 mit einem aufgeschraubten Innenadapter 4 durch das Loch des Schaftes 1, die Befestigung des Innenadapters 4 mit dem Verbinder 3 mittels eines Außenadapters 5 auf dem Verbinder 3 und somit die Aufnahme des Innenadapters 4 im Schaft 1 mit dem nach außen führenden Verbinder 3.

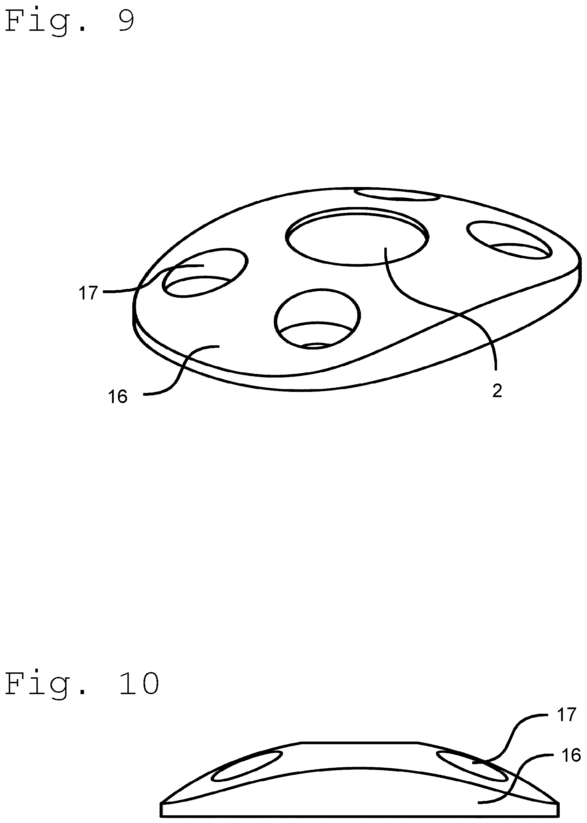

Wie oben beschrieben, wird ähnlich eine Unterarmprothese hergestellt. Hierzu werden jedoch die Dummys 16 nach den Figuren 9 und 10 verwendet. Alle Arbeitsschritte wie bei einer Oberarmprothese sind auch bei einer Unterarmprothese notwendig.

### Zusammenstellung der Bezugszeichen

- 1 -: Schaft
- 2 -: Öffnung, Aufnahme
- 3 -: Gewindestange, Verbinder
- 4 -: Innenadapter
- 5 -: Kontermutter, Außenadapter
- 6 -: Halbschale
- 7 -: Ellbogengelenk
- 8 -: Handgelenk
- 9 -: Schnittdarstellung eines Gelenkteiles
- 10 -: Nut
- 11 -: elastischer Ring
- 12 -: Schicht aus kohlenstofffaserverstärktem Kunststoff
- 13 -: Bohrungen
- 14 -: Messinggewindehülsen
- 15 -: Sicherungsschrauben, Innensechskantschrauben
- 16 -: Verstärkungselement, Dummy
- 17 -: Durchgangslöcher
- 18 -: Ansatz
- 19 -: Handprothese

## Patentansprüche

1. Armprothese in Leichtbauweise, bei hoher Stabilität und einem geringeren Gewicht gegenüber bisher bekannten Lösungen,
**dadurch gekennzeichnet,**
**dass** am distalen Ende eines an sich bekannten Schaftes (1) eine Ausbuchtung für die Aufnahme eines Innenadapters (4) mit einer Öffnung (2) als Aufnahme (2) für einen Verbinder (3) in Form einer Gewindestange (3) angeordnet ist und die Gewindestange (3) innerhalb des Schaftes (1) in dem auf der Gewindestange (3) aufgeschraubten Innenadapter (4) endet, die Gewindestange (3) durch ein Loch im Schaft (1) nach außen geführt ist und der Innenadapter (4) auf der Seite, die an der Schaftinnenseite anliegt, dem Schaft (1) um das Loch herum angepasst ist und auf der Gewindestange (3) außerhalb des Schaftes (1) eine Kontermutter (5) bzw. ein Außenadapter (5) angeordnet ist, dessen Seite zum Schaft (1) hin als Halbschale (6) ausgeführt und am anderen Ende des Verbinders (3) ein Ellbogengelenk (7) angeordnet ist, welches an beiden Enden ein Innengewinde für die Aufnahme je eines Verbinders (3), den Verbinder (3) vom Schaft (1) und einen Verbinder (3) zu einem Handgelenk (8), besitzt und das Handgelenk (8) an einer Seite ein Innengewinde zur Aufnahme des Verbinders (3) vom Ellbogengelenk (7) besitzt und am anderen Ende das Handgelenk (8) einen Ansatz (17) mit einem Außengewinde für die Aufnahme einer Handprothese (19) besitzt, wobei alle Einzelteile der Armprothese aus Kunststoff bestehen.

2. Armprothese nach Anspruch 1,
**dadurch gekennzeichnet,**
**dass** am distalen Ende des Schaftes (1) in einer zusätzlichen Ausbuchtung des Schaftes (1) um das Loch des Schaftes ein Verstärkungselement (16), ein Dummy (16) eingearbeitet ist.

3. Armprothese nach einem der vorhergehenden Ansprüche,
**dadurch gekennzeichnet,**
**dass** der Innenadapter (4) auf der Seite in Richtung Schaft (1) der Oberfläche des Schaftes (1) angepasst ist und auf der Oberfläche in Richtung Schaft (1) eine kreisförmige Nut (10) und darin eingelegt einen elastischen Ring (11) besitzt, der mit seiner Oberfläche über die Oberfläche des Innenadapters (4) hinausragt.

4. Armprothese nach einem der vorhergehenden Ansprüche,
**dadurch gekennzeichnet,**
**dass** der Außenadapter (5) auf der Seite in Richtung Schaft (1) die Form einer Halbschale (6) besitzt.

5. Armprothese nach einem der vorhergehenden Ansprüche,
**dadurch gekennzeichnet,**
**dass** die Oberfläche des Innenadapters (4) und des Außenadapters (5) in Richtung Schaft (1) elastisch ausgeführt ist oder einen elastischen Überzug oder elastische Flächen besitzt.

6. Armprothese nach einem der vorhergehenden Ansprüche,
**dadurch gekennzeichnet,**
**dass** die Verbinder (3) als Rohr ausgebildet sind und auf der Innenseite der Rohre eine Schicht (12) oder ein Rohr aus kohlenstofffaserverstärktem Kunststoff aufgetragen oder eingearbeitet ist.

7. Armprothese nach einem der vorhergehenden Ansprüche,
**dadurch gekennzeichnet,**
**dass** die Adapter (4 und 5) und die Gelenke (7 und 8) zu den Verbindern (3) mittels Sicherungsschrauben / Innensechskantschrauben (15) gesichert sind und in die dazu notwendigen Bohrungen (13) Messinggewindehülsen (14) eingearbeitet sind.

8. Verfahren zur Herstellung eines Armprothesenschaftes für die Aufnahme eines Verbinders mit Innen- und Außenadapter,
**dadurch gekennzeichnet,**
**dass** vom gesunden Arm die Maße aufgenommen werden, danach der Armstumpf mit Frischhaltefolie umwickelt wird und anschließend die Umfangsmaße des Armstumpfes aufgenommen werden, anschließend der Armstumpf mit Gipsbinden umwickelt wird und dieses Wickelmuster nach dem Aushärten vom Armstumpf abgenommen und mit Gips gefüllt wird, nach dem Aushärten dieser Gipsfüllung wird diese Gipsform modelliert und ein Innenschaft z. B. durch ein Tiefziehverfahren erzeugt, auf diesen Innenschaft wird ein Dummy aufgesetzt und mittels Knetsilikon werden die Übergänge ausgeglichen, die Durchgangslöcher (17) des Dummys (16) ausgefüllt und anschließend erfolgt unter Nutzung des Innenschaftes mit dem aufgesetzten Dummy (16) das Tiefziehen und Entformen eines Schaftes (1) und die Einarbeitung eines Lochs in den Schaft (1) am distalen Ende des Schaftes (1) und die Einführung eines Verbinders (3) mit einem aufgeschraubten Innenadapter (4) durch das Loch des Schaftes (1) und somit die Aufnahme des Innenadapters (4) im Schaft (1) mit nachfolgender Befestigung des Innenadapters (4) am Schaft (1) durch einen aufgeschraubten Außenadapter (5) auf den Verbinder (3), wobei für die Herstellung eines Oberarmschaftes die Dummys (16) nach den Figuren 1 bis 3 verwendet werden.

9. Verfahren zur Herstellung eines Unterarmprothesenschaftes für die Aufnahme eines Verbinders mit Innen- und Außenadapter,
**dadurch gekennzeichnet,**
**dass** die Arbeitsschritte gemäß Anspruch 8 auch identisch bei einer Unterarmprothesenherstellung erfolgen, hier jedoch die Dummys (16) nach den Figuren 9m und 10 verwendet werden.
